# EUROPÄISCHE PATENTSCHRIFT

(11) **EP 0 411 473 B1**
(45) Veröffentlichungstag und Bekanntmachung des Hinweises auf die Patenterteilung: **04.10.1995**
(21) Anmeldenummer: 90114317.2
(22) Anmeldetag: 26.07.1990
(51) Int. Cl.: B06B 1/06, B06B 1/02

(54) **Ultraschallerzeuger mit einem piezoelektrischen Wandler**
Ultrasonic generator with a piezoelectric transducer
Générateur à ultrason avec un transducteur piézo-électrique

(30) Priorität: 01.08.1989 DE 3925459
(43) Veröffentlichungstag der Anmeldung: 06.02.1991
(73) Patentinhaber: Ferton Holding, CH-2800 Delmont (CH)
(72) Erfinder: Abel, Martin, D-5000 Köln 30 (DE)
(74) Vertreter: Gauger, Hans-Peter, Dipl.-Ing.

(56) Entgegenhaltungen:
- EP-A- 0 272 244
- EP-A- 0 308 662
- EP-A- 0 319 631
- US-A- 4 525 790
- US-A- 4 559 826
- ULTRASONICS, Band 23, Nr. 4, Juli 1985, Seiten 151-156, Guildford, Surrey, GB; A. RAMOS-FERNANDEZ et al.: "Automatic system for dynamic control of resonance in high power and high Q ultrasonic transducers"

## Beschreibung

Die Erfindung bezieht sich auf einen Ultraschallerzeuger mit einem elektromechanischen Wandler der durch den Oberbegriff des Patentanspruchs 1 angegebenen Gattung.

Bei einem aus der DE 36 41 058 A1 bekannten Ultraschallerzeuger dieser Art ist ein magnetostriktiver Wandler verwendet, dessen Erregerspule im Kollektorkreis eines Transistors liegt. Der Emitterkreis des Transistors ist über einen zur Erfassung des Stromdurchgangs durch die Erregerspule angeordneten Widerstand und eine Spannungsauswerteschaltung mit der die Lieferung der Erregerimpulse an die Basis des Transistors vermittelnden Steuereinrichtung verbunden. Die Spannungsauswerteschaltung liefert ein digitales Signal entsprechend der an dem Widerstand abfallenden Spannung und ist entweder mit einer Reihenschaltung eines Spannungs-Frequenz-Wandlers und eines Zählers oder mit einer ebenfalls in Reihe geschalteten Anordnung einer Gleichspannungsunterdrückungsschaltung, einer Tiefpaßschaltung und eines Analog-Digital-Wandlers gebildet. Die Steuereinrichtung ist ausgangsseitig mit einem Zähler verbunden, der Impulse mit einer durch das Liefersignal der Spannungsauswerteschaltung bestimmten Impulsrate abgibt. Die Weiterleitung dieser Impulse an die Basis des Transistors ist durch einen getakteten Schaltregler festgelegt, der mit der Steuereinrichtung gesteuert wird. Die Steuereinrichtung ist noch mit einem Potentiometer verbunden, um die Leistungsvorgabe des magnetostriktiven Wandlers verändern zu können.

Bei diesem bekannten Ultraschallerzeuger mit einem magnetostriktiven Wandler ist die Steuereinrichtung ausschließlich zur Einstellung der Impulsrate der Erregerimpulse auf den Resonanz-Arbeitspunkt des Wandlers vorgesehen. Dabei werden während einer primären Abgleichphase der Schaltungsanordnung der Erregerspule Impulse mit einer jeweils vorgegebenen Anzahl von Impulsraten in jeweils aufeinanderfolgend kleineren Impulsratenbereichen angeliefert. Jeder zu Beginn der Inbetriebnahme somit in einzelnen Abgleichstufen fest eingestellten Impulsratenbereich der Erregerimpulse, bei dem der magnetostriktive Wandler mit einer Resonanzfrequenz arbeitet, wird im übrigen nur mit einer einfachen Fehleranzeige überwacht, die mit der Steuereinrichtung gesteuert wird, um bei einem Abweichen dieser Resonanzfrequenz von dem fest eingestellten Impulsratenbereich die bloße Anzeige eines vorhandenen Fehlers zu erhalten, ohne daß dabei die eigentliche Fehlerquelle gleichzeitig korrigiert wird.

Der Erfindung liegt die Aufgabe zugrunde, einen Ultraschallerzeuger der durch den Patentanspruch 1 angegebenen Gattung derart auszubilden, daß bei Verwendung eines piezoelektrischen anstelle eines magnetostriktiven Wandlers für die Leistungsabgabe eine weniger kritische Abhängigkeit von Toleranzen der mit dem Wandler verbundenen Bauteile der Schaltungsanordnung besteht und die Leistungsabgabe auch korrigierbar ist, wenn sich der während einer Einmessphase eingestellte Resonanz-Arbeitspunkt des Wandlers im Betrieb des Ultraschallerzeugers verändern sollte.

Die mit einem Ultraschallerzeuger der erfindungsgemäßen Ausbildung mit den Merkmalen nach dem Kennzeichen des Patentanspruches 1 erzielbaren Vorteile bestehen im wesentlichen darin, daß durch die mit dem Mikrocomputer realisierte ständige Nachregelung des Resonanz-Arbeitspunktes des Ultraschallerzeugers jetzt alle die Leistungsabgabe des Wandlers beeinflussenden veränderlichen Toleranzen der Schaltungsanordnung optimal aufeinander abgestimmt werden können und darüber hinaus jede Veränderung der Leistungsabgabe des Wandlers für eine optimale Beibehaltung des Resonanz-Arbeitspunktes kontinuierlich berücksichtig werden kann. Die Verwendung eines Mikrocomputers ergibt dabei gleichzeitig noch den wesentlichen Vorteil, daß der Gerätehersteller umschaltbare Einzelprogramme mit unterschiedlichen Leistungsvorgaben eiplanen kann. Es wird dann ein entsprechend universell einsetzbarer Ultraschallerzeuger bereit gestellt, der benutzerseitig ein unproblematisches Arbeiten als Folge eines vorhandenen Einzelprogramms für jede spezielle Arbeitsfolge erlaubt.

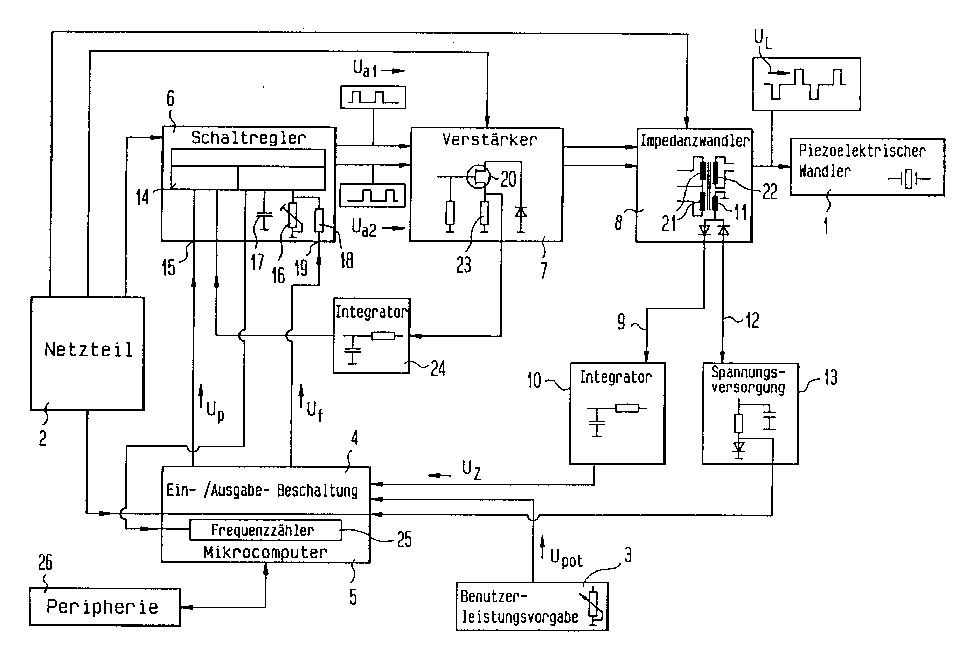

Ein Ausführungsbeispiel des erfindungsgemäßen Ultraschallerzeugers ist in der Zeichnung mit einem Blockschaltbild schematisch dargestellt und wird nachfolgend näher erläutert.

Der Ultraschallerzeuger ist mit einem piezoelektrischen Wandler 1 gebildet, dessen Treiberkreis aus einem mit Wechselspannung versorgten Netzteil 2 mit einer Arbeitsspannung U_{L} versorgt wird. Das Netzteil 2 ist mit einem Gleichrichter und einem Glättungskondensator ausgebildet. Wenn der Ultraschallerzeuger zur Verwendung bei einem Gerät zur Zahnbehandlung vorgesehen ist, so befindet sich dabei der piezoelektrische Wandler 1 in einem über eine Kabelverbindung an das Gerät angeschlossenen Handstück, um an einer beispielsweise für eine Zahnsteinentfernung oder auch für eine Wurzelbehandlung speziell ausgebildeten Instrumentenspitze den mit dem Wandler übersetzten Ultraschall für die Zahnbehandlung zur Anwendung zu bringen. Für die Zahnbehandlung ergeben sich dabei unterschiedliche Leistungsvorgaben für den sogenannten Resonanz-Arbeitspunkt des Wandlers, die für eine angepaßte Veränderungsmöglichkeit seiner Arbeitsspannung U_{L} mit einem Potentiometer 3 anwählbar sind.

Der Abgriff des Potentiometers 3 ist mit der Ein-/Ausgabe-Beschaltung 4 eines aus dem Netzteil 2 versorgten Mikrocomputers 5 verbunden. Der Mikrocomputer 5 ergibt eine mit dem piezoelektrischen Wandler 1 rückgekoppelte Steuereinrichtung, die einen aus dem Netztteil 2 in einer Kaskadenschaltung versorgten Schaltregler 6 beispielsweise des Typs TL 494 von Texas Instruments steuert. Der Schaltregler 6 ist über einen Verstärker 7 mit einem Impedanzwandler 8 verbunden, die beide aus dem Netzteil 2 mit einer gegenüber dem Mikrocomputer 5 und dem Schaltregler 6 wesentlich höheren Versorgerspannung versorgt werden. Der Impedanzwandler 8 ist durch einen Transformator bereitgestellt, der die Arbeitsspannung U_{L} für den piezoelektrischen Wandler 1 liefert.

Der piezoelektrische Wandler 1 ist mit dem Mikrocomputer 5 über eine mit dem Impedanzwandler 8 verbundene Impedanz-Meßschaltung 9 rückgekoppelt. Die Impedanz-Meßschaltung 9 umfaßt einen als Tiefpaß ausgebildeten Integrator 10 und ist an eine Hilfswicklung 11 des Impedanzwandlers 8 angeschlossen. Die magnetische Kopplung des Impedanzwandlers 8 ist dadurch für eine entsprechend präzise Bestimmung des Istwertes der Impedanz des gesamten Treiberkreises des piezoelektrischen Wandlers 1 entsprechend optimal erfaßbar. Mit der Impedanz-Meßschaltung 9 wird dem Mikrocomputer 5 ein Meßwert für die Spannung U_{Z} entsprechend dem damit ermittelten Istwert der Impedanz zugeführt, so daß mit diesem Meßwert ein ständiger Ist-/Soll-Vergleich durchgeführt werden kann. An der Hilfswicklung 11 wird im übrigen die Betriebsspannung für die Ein-/Ausgabe-Beschaltung 4 des Mikrocomputers 5 abgenommen, wobei in der betreffenden Versorgungsleitung 12 ein Spannungsregler 13 angeordnet ist.

Der mit dem Mikrocomputer 5 gesteuerte Schaltregler 6 befindet sich auf der Sekundärseite des Netztransformators. Die unter Vermittlung des Gleichrichters des Netzteils 2 zugeführte Gleichspannung wird durch den Schaltregler 6 in eine Wechselspannung umgewandelt, deren Tastverhältnis die dem Verstärker 7 zugeführte Arbeitsspannung U_{L} bestimmt. Das Tastverhältnis wird durch einen Regelverstärker 14 festgelegt, der an einem Regeleingang 15 des Schaltreglers 6 mit der Ein-/Ausgabe-Beschaltung 4 des Mikrocomputers 5 verbunden ist. Das Tastverhältnis kann daher durch den Mikrocomputer 5 gesteuert werden, wobei die maßgebliche Regelspannung Uₚ aus der als Sollwert für den Arbeitspunkt des piezoelektrischen Wandlers 1 übersetzten Spannung Uₚₒₜ der Leistungsvorgabe erhalten wird. Die Frequenz der Ausgangsspannung des Spannungsreglers 6 wird andererseits mit einem Trimmwiderstand 16 und einem Kondensator 17 erhalten, die beide über einen frequenzmodulierenden Vorschaltwiderstand 18 an einem Steuereingang 19 des Schaltreglers 6 liegen. Auch der Steuereingang 19 ist mit der Ein-/Ausgabe-Beschaltung 4 des Mikrocomputers 5 verbunden, womit auch die Frequenz der Ausgangsspannung mit einer durch den Mikrocomputer vorgegebenen Steuerspannung U_{f} in der Größenordnung zwischen 0 und 2.5 V auf einen von dem Resonanz-Arbeitspunkt des Wandlers abhängigen zeitlichen Mittelwert gesteuert werden kann.

Der Verstärker 7 ist bevorzugt mit zwei im Gegentakt arbeitenden Transistoren ausgebildet, um für den piezoelektrischen Wandler eine Begrenzung seiner Arbeitsspannung U_{L} auf etwa 300 V zu erhalten. Die Verwendung nur eines Transistors 20 für den Verstärker 7 würde eine Arbeitsspannung von etwa 600 V ergeben, so daß mit dieser Gegentaktstufe neben einer verbesserten Aussteuerbarkeit hauptsächlich ein Depolarisationsschutz für den piezoelektrischen Wandler erhalten wird. Als Transistoren werden Feldeffekt-Transistoren bevorzugt, denen die getakteten Ausgangsspannungen Uₐ₁ und Uₐ₂ des Schaltreglers 6 abwechselnd angeliefert werden. Nach der Verstärkung durch den Verstärker 7 werden die getakteten Ausgangsspannungen des Schaltreglers 6 an zwei Primärwicklungen 21 des Impedanzwandlers 8 angeliefert, um durch dessen Sekundärwicklung 22 zu der Arbeitsspannungen U_{L} transformiert zu werden. Die Verstärkung der Ausgangsspannung des Schaltreglers 6 ist noch dadurch verfeinert, daß der Verstärkerstrom an einem an Masse liegenden Widerstand 23 abgenommen und über einen als Tiefpaß ausgebildeten Integrator 24 über den Regeleingang 15 des Schaltreglers 6 dem Regelverstärker 14 zugeführt ist. Damit wird für die Steuerung des Tastverhältnisses des Schaltreglers 6 eine zur Konstanthaltung des Verstärkerstroms genutzte Rückkoppelung erhalten. Diese Rückkoppelung ist über eine Verbindung des Schaltreglers 6 mit einem Frequenzzähler 25 des Mikrocomputers 5 noch weiter verfeinert, so daß damit alle Möglichkeiten der Schaltungsanordnung genutzt sind, um den Resonanz-Arbeitspunkt des piezoelektrischen Wandlers 1 optimal bestimmen und auch ständig überwachen zu können.

Für die Arbeitsweise des vorbeschriebenen Ultraschallerzeugers in der Verwendung bei einem Gerät zur Zahnbehandlung mittels Ultraschall ist damit im wesentlichen von den folgenden Gegebenheiten auszugehen. Nach dem Einschalten des Gerätes wird zunächst durch den Mikrocomputer 5 eine von einer Leistungsvorgabe mittels des Potentiometers 3 abhängige Frequenz-Impedanz-Analyse für den in das Handstück eingebauten piezoelektrischen Wandler 1 aktualisiert. Es wird dafür die mit der Impedanz-Meßschaltung 9 an der Hilfswicklung 11 des Impedanzwandlers 8 erfaßte Impedanz als eine den Resonanz-Arbeitspunkt des piezoelektrischen Wandlers 1 respektive dessen Serien-Resonanzfrequenz somit beeinflussende Absolutgröße der entsprechenden Spannung U_{Z} mittels des Mikrocomputers 5 bestimmt. Die Bestimmung führt dabei zu der Ermittlung eines Sollwertes für die Steuerspannung U_{f}, mit welcher somit die Arbeitsspannung U_{L} des piezoelektrischen Wandlers 1 festgelegt wird. Für einen vorgegebenen Resonanz-Arbeitspunkt des piezoelektrischen Wandlers 1 liegt dessen Arbeitsspannung U_{L} andererseits bei einer Frequenz, bei welcher die für einen piezoelektrischen Wandler typische Impedanzkurve einen plötzlichen Abfall zu einem Tiefwert ergibt, der nach Überschreitung der somit für den Resonanz-Arbeitspunkt des Wandlers vorbestimmten Frequenz sofort wieder verlassen wird. Bei dieser primären Ermittlung des Sollwertes für die $teuerspannung U_{f} und somit eines vorbestimmten Resonanz-Arbeitspunktes des piezoelektrischen Wandlers 1 wird auch der Verstärkerstrom durch die über den Widerstand 23 und den Integrator 24 vermittelte Rückmeldung an den Regelverstärker 14 des Schaltreglers 6 sowie auch die weitere Rückmeldung an den Frequenzzähler 25 des Mikrocomputers 5 berücksichtigt.

Sobald mit dem Handstück des Gerätes gearbeitet wird, wird durch die Impedanz-Meßschaltung 9 eine ständige Rückmeldung an den Mikrocomputer 5 vorgenommen, um einen Ist-/Soll-Vergleich mit der an dem Potentiometer 3 vorgewählten Leistungsvorgabe durchzuführen. Jede bei diesem ständigen Vergleich festgestellte Abweichung löst ein Nachsteuern des Schaltreglers 6 entweder an dem Regeleingang 15 für eine Anpassung des Tastverhältnisses oder/und an dem Steuereingang 19 für einen Anpassung der Arbeitsspannung U_{L} des piezoelektrischen Wandlers 1 aus.

Die Eingliederung des Mikrocomputers 5 für die Steuerung des Schaltreglers 6 gibt daneben auch die Möglichkeit des Anschlusses eines externen Peripherie-Gerätes 26 an eine Schnittstelle des Mikrocomputers, so beispielsweise eines für Servicezwecke vorgesehenen Personal-Computers. Auch kann damit für den Ultraschallerzeuger ein Arbeitsprogramm bereitgestellt werden, mit dem die unterschiedlichen Leistungsvorgaben beispielsweise für eine mit dem Handstück durchführbare Zahnsteinentfernung einerseits und eine mit einer ausgewechselten Instrumentenspitze des Handstückes durchführbare Wurzelbehandlung andererseits berücksichtigt sind. Die Berücksichtigung wird dabei mit Erfahrungswerten zum Erreichen einer jeweils optimalen Zahnbehandlung mit den in Frage kommenden unterschiedlichen Instrumentenspitzen gestützt, so daß bei einem Arbeiten mit dem Gerät nicht mehr mit individuellen Leistungsvorgaben experimentiert werden muß.

Der sekundärgetaktete Schaltregler 6, der im Zusammenwirken mit dem Transformator als ein Aufwärts-Wandler ausgebildet ist, kann auch durch einen primärgetakteten Schaltregler ersetzt sein. Zur Netztrennung müßte dann anstelle des normalen Netztransformators bei dem Netzteil 2 ein Hochfrequenztransformator eingesetzt werden. Weiterhin kann die an die Hilfswicklung 11 des Impedanzwandlers 8 angeschlossene Impedanz-Meßschaltung alternativ auch einen Anschluß an die Primärwicklung 21 oder auch an die Sekundärwicklung 22 des Impedanz-Wandlers aufweisen. Sofern die Messung des Verstärkerstroms realisiert ist, kann als weitere Alternative schließlich auch eine Meßschaltung vorgesehen werden, die nicht mehr an den Impedanzwandler 8, sondern vielmehr an die Ausgangsseite des Integrators 24 angeschlossen ist.

## Patentansprüche

1. Ultraschallerzeuger insbesondere zur Verwendung bei einem Gerät zur Zahnbehandlung mittels Ultraschall mit:
einem piezoelektrischen Wandler (1), dessen Treiberschaltkreis (6, 7, 8) einen über einen Oszillator (6) gespeisten Verstärker (7) und einen damit seriell verbundenen Impedanzwandler (8) umfaßt;
einer Steuereinrichtung (5), die das Tastverhältnis und die Frequenz des Oszillators (6) steuert, indem sie ständig einen Ist-/Soll-Vergleich zwischen der für den piezoelektrischen Wandler (1) mittels einer Impedanzmeßschaltung (9) erfaßten Impedanz als Istwert und einem Sollwert durchführt, und die bei jeder gegenüber dem Sollwert festgestellten Abweichung eine Anpassung des Tastverhältnisses mittels einer einem Regeleingang (15) des Oszillators (6) zugeführten Regelspannung (Up) und/oder eine Anpassung der Frequenz mittels einer einem Steuereingang (19) des Oszillators (6) zugeführten Steuerspannung (Uf) steuert, dadurch **gekennzeichnet**, daß
der Oszillator einen analogen getakteten Schaltregler (6) umfaßt;
die Leistung für den piezoelektrischen Wandler (1) mittels eines Potentiometers (3) veränderbar vorgegeben werden kann;
die Regel- und Steuereingänge (15, 19) des genannten Schaltreglers (6) sowie die Impedanzmeßschaltung (9) und das Potentiometer (3) an eine Ein-/Ausgabe-Beschaltung (4) der als Mikrocomputer ausgebildeten Steuereinrichtung (5) angeschlossen sind; und
der Schaltregler (6) mit einem Frequenzzähler (25) im Mikrocomputer (5) rückgekoppelt ist und dieser neben der genannten Impedanz auch die mit dem Frequenzzähler (25) gemessene Frequenz mit einem Sollwert vergleicht, der sich aus der Leistungsvorgabe für den Resonanzarbeitspunkt, gemessener Impedanz und Frequenz errechnet.

2. Ultraschallerzeuger nach Anspruch 1,
dadurch **gekennzeichnet,** daß der Verstärker (7) mit einem Konstantstrom arbeitet und der Verstärkerstrom an einem an Masse liegenden Widerstand (23) des Verstärkers (7) abgenommen und über einen als Tiefpaß ausgebildeten Integrator (24) einem zweiten Regeleingang des Schaltreglers (6) zugeführt wird.

3. Ultraschallerzeuger nach Anspruch 1 oder 2,
dadurch **gekennzeichnet,** daß an eine Schnittstelle des Mikrocomputers (5) ein externes Peripheriegerät (26) angeschlossen ist.

4. Ultraschallerzeuger nach Anspruch 3,
dadurch **gekennzeichnet,** daß das an die Schnittstelle des Mikrocomputers (5) angeschlossene Peripheriegerät (26) ein für Servicezwecke vorgesehener Personalcomputer ist.

5. Ultraschallerzeuger nach einem der Ansprüche 1 bis 4,
dadurch **gekennzeichnet,** daß der Mikrocomputer (5) mit einem Arbeitsprogramm ausgerüstet ist, bei dem mit unterschiedlichen Leistungsvorgaben unterschiedliche Resonanzarbeitspunkte des piezoeelektrischen Wandlers berückichtigt sind, wobei eine Umschaltmöglichkeit zwischen den verschiedenen Einzelprogrammen für jeweils eine vorherbestimmte Leistungsvorgabe vorgesehen ist.

6. Ultraschallerzeuger nach einem der Ansprüche 1 bis 5,
dadurch **gekennzeichnet,** daß die Impedanzmeßschaltung (9) an eine Hilfswicklung (11) des Impedanzwandlers (8) angeschlossen ist.

7. Ultraschallerzeuger nach einem der Ansprüche 1 bis 6,
dadurch **gekennzeichnet,** daß der Verstärker (7) mit zwei im Gegentakt arbeitenden Transsistoren ausgebildet ist.

## Claims

1. An ultrasonic converter in particular for use in a device for treating teeth by means of ultrasonics, comprising:
a piezoelectric converter (1) whose driver circuit (6, 7, 8) comprises an amplifier (7) supplied via an oscillator (6), and an impedance converter (8) connected in series therewith;
a control means (5) for controlling the pulse-duty factor and the frequency of said oscillator (6) by performing a permanent actual/reference comparison between the impedance as a respective actual value as detected by means of an impedance-measuring circuit (9) for said piezoelectric converter (1) and a corresponding reference value, and which upon any detection of a deviation from said reference value controls an adaptation of the pulse-duty factor by means of a regulation voltage (Up) supplied to a regulating input (15) of said oscillator (6) and/or an adaptation of the frequency by means of a control voltage (Uf) supplied to a control input (19) of the oscillator (6), characterized in that said oscillator comprises a switching regulator (6) being analog timed;
the power for said piezoelectric converter (1) being adapted to be variably preset by means of a potentiometer (3);
said regulating and control inputs (15, 19) of said switching regulator (6) as well as said impedancemeasuring circuit (9) and said potentiometer (3) being connected to an input/output wiring (4) of said control means (5) operating as a microcomputer; and
said switching regulator (6) having a feedback connection with a frequency counter (25) included in said microcomputer (5) which besides of said impedance also compares the frequency measured by said frequency counter (25) with a corresponding reference value which is calculated from said preset power for the resonance operating point, the measured impedance and the frequency.

2. An ultrasonic generator according to claim 1,
characterized in that said amplifier (7) operates on a constant current and the amplifier current is tapped at a grounded resistor (23) of said amplifier (7) and supplied to a second regulating input of said switching regulator (6) through and integrator (24) implemented as a low-pass device.

3. An ultrasonic generator according to claim 1 or 2,
characterized in that an external peripheral equipment (26) is connected to an interface of said microcomputer (26).

4. An ultrasonic generator according to claim 3,
characterized in that said peripheral equipment (26) connected to the interface of said microcomputer (5) is a personal computer for servicing purposes.

5. An ultrasonic generator according to any of the claims 1 to 4,
characterized in that said microcomputer (5) is provided with an operating program which considers different preset powers for different resonance operating points of said piezoelectric converter whereby provision is made for a possible changeover between the various individual programs each for a respective predetermined power presetting.

6. An ultrasonic generator according to any of the claims 1 to 5,
characterized in that said impedance-measuring circuit (9) is connected to an auxiliary coil (11) of said impedance converter (8).

7. An ultrasonic generator according to any of the claims 1 to 6,
characterized in that said amplifier (7) is provided with two transistors operating in a push-pull mode.

## Revendications

1. Générateur d'ultrasons, en particulier pour une application dans un dispositif de soins dentaires aux ultrasons, comprenant:
un convertisseur piézoélectrique (1), dont le circuit d'attaque (6, 7, 8) comprend un amplificateur (7), alimenté via un oscillateur (6), ainsi qu'un adaptateur d'impédance (8) y branché en série;
un organe de commande (5) qui commande le rapport durée de passage de courant/durée de cycle et la fréquence dudit oscillateur (6) moyennant un comparaison permanente des chiffres réels avec les chiffres prévus entre une impédance détectée, moyennant un montage à mesurer l'impédance (9), comme valeur réelle et une valeur de consigne, et qui, au cas de tout écart relatif à la valeur de consigne, commande une adaptation dudit rapport durée de passage de courant/durée de cycle moyennant une tension d'asservissement (Up), alimentée à une entrée d'asservissement (15) dudit oscillateur (6), et/ou une adaptation de la fréquence moyennant une tension de commande (Uf) alimentée à l'entrée de commande (19) dudit oscillateur (6),
**caractérisé** en ce que
ledit oscillateur comprend un régulateur de commande rythmé analogique (6);
en ce qu'on peut déterminer le débit dudit convertisseur piézoélectrique (1) de façon variable moyennant un potentiomètre (3);
en ce que lesdites entrée d'asservissement et de commande (15, 19) dudit régulateur de commande (6), ainsi que ledit montage à mesurer l'impédance (9) et ledit potentiomètre (3) sont raccordée à un circuit d'entrée/sortie (4) dudit organe de commande (5), qui est conçu sous forme d'un micro-ordinateur; et
en ce que ledit régulateur de commande (6) est connecté, à réinjection, à un compteur de fréquence (25) dans ledit microordinateur (5), et que le dernier établit une comparaison entre non seulement ladite impédance mais aussi la fréquence mesurée moyennant ledit compteur de fréquence (25), d'une part, et une valeur de consigne, d'autre part, qui est calculée à la base du débit spécifié pour le point de fonctionnement en résonance, ainsi qu'à la base de l'impédance mesurée et de la fréquence.

2. Générateur d'ultrasons selon la revendication 1,
**caractérisé** en ce que ledit amplificateur (7) fonctionne à courant constant et le courant dudit amplificateur (7) est prélevé à un résisteur (23), mis à la terre, dudit amplificateur (7) et est alimenté à une deuxième entrée d'asservissement dudit régulateur de commande (6) via un intégrateur (24) sous forme d'un filtre passe-bas.

3. Générateur d'ultrasons selon la revendication 1 ou 2,
**caractérisé** en ce qu'une unité périphérique (26) extérieure est raccordée à un interface dudit micro-ordinateur (5).

4. Générateur d'ultrasons selon la revendication 3,
**caractérisé** en ce que ladite unité périphérique (26) raccordée à l'interface dudit micro-ordinateur (5) est un ordinateur personnel à des fins de service après-vente.

5. Générateur d'ultrasons selon une quelconque des revendications 1 à 4,
**caractérisé** en ce que ledit micro-ordinateur (5) est équipé d'un programme opérationnel, dans lequel des points de fonctionnement à résonance dudit convertisseur piézoéletrique sont pris en considération par des valeurs de débit de consigne différentes, à une possibilité étant prévue pour commuter entre les programmes individuels différents dont chacun fonctionne à une valeur de débit de consigne respectif déterminée.

6. Générateur d'ultrasons selon une quelconque des revendications 1 à 5,
**caractérisé** en ce que ledit montage à mesurer l'impédance (9) est raccordé à un bobinage auxiliaire (11) dudit adaptateur d'impédance (8).

7. Générateur d'ultrasons selon une quelconque des revendications 1 à 6,
**caractérisé** en ce que ledit amplificateur (7) est formé de façon à comprendre deux transistors en montage push-pull.
